# EUROPEAN PATENT APPLICATION

(11) **EP 4 246 143 A1**
(43) Date of publication of application: **20.09.2023**
(21) Application number: 22162413.3
(22) Date of filing: 16.03.2022
(51) Int. Cl.: G01N 33/50, G01N 33/564

(54) **IN-VITRO METHOD FOR DIAGNOSIS OF PEMPHIGUS VULGARIS AND/OR OF DETERMINING DISEASE ACTIVITY IN A PEMPHIGUS VULGARIS PATIENT**

(71) Applicant: PHILIPPS-UNIVERSITÄT MARBURG, 35037 Marburg (DE)
(72) Inventor: Hudemann, Christoph, 34131 Kassel (DE); Hertl, Michael, 35039 Marburg (DE)
(74) Representative: Durm Patentanwälte PartG mbB

(57) **Abstract**

Disclosed is an *in vitro* method for diagnosis of pemphigus vulgaris in a patient and/or of determining disease activity in a pemphigus vulgaris patient, the method comprising determination of T cells specific for the extracellular domain of desmoglein 3 (Dsg3-specific T-cells) or for specific epitopes of said extracellular Dsg3 domain in serum of said patient by HLA-class II-dextramer staining.

## Description

The present invention relates to diagnosis and determination of the disease progression in pemphigus vulgaris. In particular, it relates to an *in vitro* method for diagnosis of pemphigus vulgaris in a patient and/or of determining disease activity in a pemphigus vulgaris patient, the method comprising determination of T cells specific for the extracellular domain of desmoglein 3 (Dsg3) and/or distinct Dsg3 epitopes, preferably Dsg3₍₂₀₆₋₂₂₀₎ and Dsg3₍₃₇₈₋₃₉₂₎.

### Background

Pemphigus encompasses a group of life-threatening skin diseases, including the most common clinical variants, pemphigus vulgaris (PV) and pemphigus foliaceus (PF). PV is characterized by a loss of epidermal cell-cell adhesion which leads to progressive flaccid blisters and erosions of the mucous membranes that is directly linked to the presence of IgG autoantibodies against the desmosomal transmembrane protein, desmoglein (Dsg)3. Upon additional involvement of the skin which is commonly seen in the chronic disease course, PV sera also contain IgG against the desmosomal adhesion molecule, Dsg1.

Pemphigus encompasses a group of autoimmune skin disorders caused by autoantibodies against epidermal desmosomes.

A desmosome ("binding body"), also known as a macula adherens, is a cell structure specialized for cell-to-cell adhesion. A type of junctional complex, they are localized spot-like adhesions randomly arranged on the lateral sides of plasma membranes. Desmosomes are one of the stronger cell-to-cell adhesion types and are found in tissue that experience intense mechanical stress, such as cardiac muscle tissue, bladder tissue, gastrointestinal mucosa, and epithelia.

Desmosomes are composed of desmosome-intermediate filament complexes (DIFC), which is a network of cadherin proteins, linker proteins and keratin intermediate filaments. The DIFCs can be broken into three regions: the extracellular core region, or desmoglea, the outer dense plaque, or ODP, and the inner dense plaque, or IDP.

The extracellular core region, approximately 34 nm in length, contains desmoglein and desmocollin, which are in the cadherin family of cell adhesion proteins. Both have five extracellular domains, and have calcium-binding motifs. Extracellular calcium helps form the cadherin adhesion by allowing the cadherin extracellular domain on desmoglein and desmocollin to become rigid. They bind to each other via heterophilic interactions in the extracellular space near their N-termini, in contrast with the homophilic binding characteristic of other cadherins. Desmoglein and desmocollin have a single pass transmembrane region plus an intracellular anchor to secure its position in the cell membrane.

Pemphigus vulgaris (PV) is linked to IgG against desmoglein (Dsg)3 and Dsg1 and causes mucosal and skin blisters. Dsg-specific T cells play a central role in PV pathogenesis as they provide help to autoreactive B cells for IgG autoantibody production.

The desmogleins are a family of desmosomal cadherins consisting of proteins Dsg1, Dsg2, Dsg3, and Dsg4. They play a role in the formation of desmosomes that connect cells to one another. Desmogleins are targeted in the autoimmune disease pemphigus vulgaris (PV). The structures and sequences of these proteins are well-known and characterized.

The amino acid sequence of the extracellular part of Dsg3 (human) is depicted in SEQ ID NO:9. It was obtained from https://www.uniprot.org/uniprot/P32926 (Dsg3).

In particular, PV and PF are associated with IgG autoantibodies against two desmosomal transmembrane proteins, desmogleins Dsg3 and Dsg1, respectively. Both clinical variants are characterized by a loss of epidermal cell adhesion of stratified epithelia, which leads to progressive flaccid blisters and erosions of the mucous membranes (PV) and/or skin (PV, PF). The inventors and others have shown in PV that autoreactive T cells against Dsg3 are crucial for the development of an autoreactive B cell response in PV. They are not only involved in the initiation but also in the perpetuation of anti-Dsg3 IgG autoantibody production.

Specifically, in PV, T cell recognition of Dsg3 involves close association with two distinct human leukocyte antigen (HLA) class II alleles, i.e. HLA-DRB1*04:02 and HLA-DQB1*05:03. Distinct peptide binding motifs in the variable β1-chain of these HLA class II alleles confer selective binding of Dsg3 peptide sequences with specific positively charged amino acids. Accordingly, a set of immunodominant Dsg3 peptides that show strong binding to these HLA molecules has already been identified as relevant T cell epitopes in PV patients (M. Hertl et al., "T cell control in autoimmune bullous skin disorders", J Clin Invest 2006; 116:1159-66; 10.1172/JCI28547) and in healthy carriers of the above mentioned PV-associated HLA class II alleles (C. M. Veldman et al., "T cell recognition of desmoglein 3 peptides in patients with pemphigus vulgaris and healthy individuals", J Immunol. 2004; 172:3883-92; 10.4049/jimmunol.172.6.3883).

Moreover, immunization of HLA-DRB1*04:02-transgenic mice with a set of these Dsg3 peptides induced human pathogenic Dsg3-specific IgG antibodies, an observation supporting the current concept that priming of Dsg3-specific T cells is a prerequisite for the induction of pathogenic IgG autoantibodies against conformational epitopes of Dsg3 (R. Eming et al., "Pathogenic IgG antibodies against desmoglein 3 in pemphigus vulgaris are regulated by HLA-DRB1*04:02-restricted T cells", J Immunol. 2014; 193:4391-9; 10.4049/jimmunol.1401081).

Thus, considering their central role in pemphigus pathogenesis, autoreactive Dsg-specific T cells are considered to have major potential to act as therapeutic targets in PV (see Hertl et al., "T cell control in autoimmune bullous skin disorders", J Clin Invest. 2006 May;116(5):1159-66. doi: 10.1172/JCI28547.J Clin Invest. 2006. PMID: 16670756; Pollmann et al., "Pemphigus: a Comprehensive Review on Pathogenesis, Clinical Presentation and Novel Therapeutic Approaches", J Clin Invest 2006; Clin Rev Allergy Immunol 2018 Feb;54(1):1-25. doi: 10.1007/s12016-017-8662-z).

The object of the invention, consequently, is to provide a method of diagnosis of pemphigus vulgaris and a method of diagnosis of disease activity in a pemphigus vulgaris patient based on determination of T cells specific for distinct Dsg3-epitopes in serum of said patient.

### Description of the invention

The inventors thoroughly characterized autoreactive T cell responses in PV by means of their cytokine profile and epitope specificity. Their findings show a predominance of type 2 T cells reactive with the extracellular domain of Dsg3 (Dsg3₍₁₋₅₆₆₎) (SEQ ID NO:9) and/or with distinct epitopes thereof in PV patients.

Autoreactive T cells that recognized a set of Dsg3 epitopes were significantly increased in PV versus HC (healthy control subjects). In particular, frequencies of autoreactive T cells specific for two distinct Dsg3 epitopes, Dsg3₍₂₀₆₋₂₂₀₎ and Dsg3₍₃₇₈₋₃₉₂₎, were significantly correlated in PV patients.

Furthermore, in an HLA-DRB1*04:02-transgenic preclinical animal model of PV, immunization with the same set of Dsg3 peptides led to the induction of pathogenic anti-Dsg3 IgG antibodies. These findings strongly suggest that T cells specific for distinct epitopes of the Dsg3 ectodomain are critical for the induction and maintenance of a rather polyclonal pathogenic IgG autoantibody response against Dsg3 in PV.

The inventors characterized Dsg3-specific peripheral T cells in a cohort of 52 PV patients and 41 healthy control subjects (HC) with regard to cytokine profile and epitope specificity. By *ELISpot analysis*, type 2 T cells reactive against the Dsg3 ectodomain (i.e. the extracellular domain) were significantly increased in PV patients compared to HC. By *Dextramer*^{®} analysis, CD4⁺ T cells specific for a set of epitopes within the extracellular domain of Dsg3 were found at significantly higher frequencies in PV patients than in HLA-matched HC. T cell recognition of two distinct Dsg3 epitopes, i.e. Dsg3₍₂₀₆₋₂₂₀₎ and Dsg3₍₃₇₈₋₃₉₂₎, correlated significantly suggesting a synergistic effect in B cell help. Immunization of HLA-DRB1*04:02-transgenic PV mice with the same set of Dsg3 peptides induced pathogenic Dsg3-specific IgG antibodies which induced loss of keratinocyte adhesion in vitro.

Thus, Dsg3 peptide-specific T cells are of particular interest as surrogate marker of disease activity and potential therapeutic targets in PV.

ELISpot (enzyme-linked immune absorbent spot) is a type of assay that focuses on quantitatively measuring the frequency of cytokine secretion for a single cell. The ELISpot Assay is also a form of immunostaining since it is classified as a technique that uses antibodies to detect a protein analyte, with the word analyte referring to any biological or chemical substance being identified or measured.

Dextramer^{®} is a technology offered by Immudex, Denmark, involving specific reagents which contain multiple binding sites and numerous fluorophores to boost avidity in binding antigen-specific T cells to isolate populations even when these are rare or have low-affinity receptors.
1. An *in vitro* method for diagnosis of pemphigus vulgaris in a patient and/or of determining disease activity in a pemphigus vulgaris patient, the method comprising determination of T cells specific for the extracellular domain of desmoglein 3 (Dsg3-specific T-cells) or for specific epitopes of said extracellular Dsg3 domain in serum of said patient by HLA-class II-dextramer staining.
2. The method of (1), wherein the Dsg3-specific T cells are peripheral CD4⁺ T-cells.
3. The method of (1) or (2), additionally comprising determination of the cytokine profile of said Dsg3-specific T cells by ELISpot analysis.
4. The method of any of (1) to (3), wherein the Dsg3-specific T cells are specific for the Dsg3₍₁₋₅₆₆₎ extracellular domain having the sequence shown in SEQ ID NO:9, wherein one to three amino acids may be deleted, added or substituted by other amino acids.
5. The method of any of (1) to (4), wherein the Dsg3-specific T cells are specific for the Dsg3 epitopes Dsg3₍₁₉₀₋₂₀₄₎ having SEQ ID NO:1, Dsg3₍₂₀₆₋₂₂₀₎ having SEQ ID NO:2, Dsg3₍₂₅₁₋₂₆₅₎ having SEQ ID NO: SEQ ID NO:3, Dsg3₍₃₇₅₋₃₉₁₎ having SEQ ID NO:4, Dsg3₍₂₅₄₋₂₆₈₎ having SEQ ID NO:7 and/or Dsg3₍₃₇₈₋₃₉₂₎ having SEQ ID NO: 8, wherein, in said sequences, one to three amino acids may be deleted or added or substituted by other amino acids, respectively.
6. The method of any of (1) to (5), wherein the Dsg3-specific T cells that are specific for the Dsg3 epitopes Dsg3₍₂₀₆₋₂₂₀₎ having SEQ ID NO: 2 and Dsg3₍₃₇₈₋₃₉₂₎ having SEQ ID NO:8 correlate to each other.
7. The method of any of (1) to (6), additionally comprising a step of determining if the patients carry the HLA-class I alleles HLA-DRB1*04:02 and/or HLA-DQB1*05:03.

### Detailed description of the invention

### Peripheral blood T cell responses against the Dsg3 ectodomain in PV patients and healthy carriers of PV associated HLA class II alleles

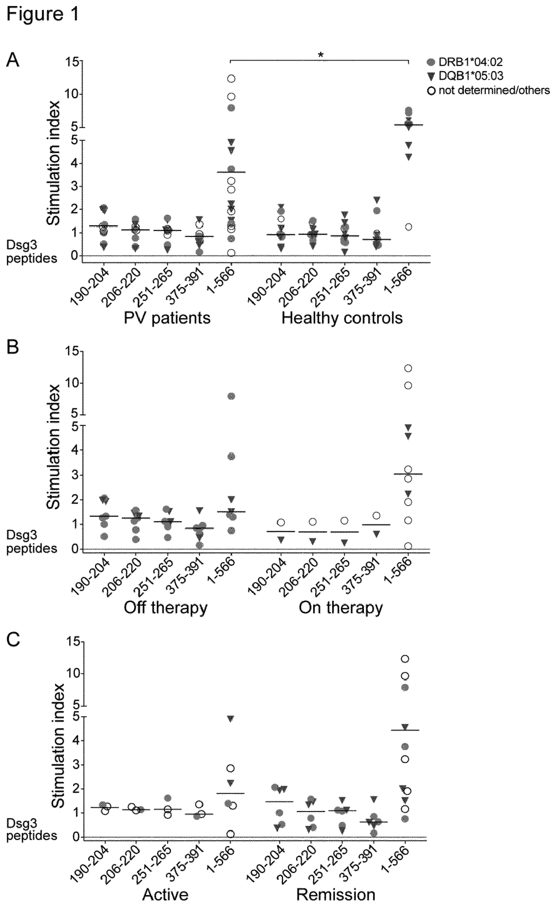

The ³H-thymidine incorporation assay has been widely used to detect T cellular proliferative responses against recombinant Dsg3 protein in human studies with PV patients and in preclinical mouse models of PV. By this approach, the inventors first sought to evaluate *ex vivo* peripheral T cell responses of PV patients against the extracellular domain of Dsg3 (Dsg3₍₁₋₅₆₆₎, SEQ ID NO:9) as well as against previously identified (R. Eming 2014; a.a.O.) immunodominant epitopes of Dsg3, i.e. Dsg3₍₁₉₀₋₂₀₄₎ (SEQ ID NO:1), Dsg3₍₂₀₆₋₂₂₀₎ (SEQ ID NO:2), Dsg3₍₂₅₁₋₂₆₅₎ (SEQ ID NO:3), Dsg3₍₃₇₅₋₃₉₁₎ (SEQ ID NO:4) (Fig. 1A-C). They observed T cell proliferative responses against Dsg3₍₁₋₅₆₆₎ (SEQ ID NO:9) in the studied PV patients and also in healthy carriers of the PV-associated HLA-alleles, HLA-DRB1*04:02 and HLA-DQB1*05:03 (Figure 1A). The latter showed a significantly higher proliferative response to Dsg3₍₁₋₅₆₆₎, presumably due to the absence of immunosuppressive treatment and a higher number of known HLA-DRB1*04:02+ donors.

In contrast, the inventors did not observe proliferative T cell responses (stimulatory index > 2) against the studied immunodominant Dsg3 peptides both in PV patients and HC. Neither were there significant differences in the proliferative T cell response against Dsg3 in PV patients on immunosuppressive therapy versus off therapy (Fig. 1B) nor in active versus remittent disease (Fig. 1C). Hence, using ³H-thymidine incorporation assay, they could not detect quantitative differences in Dsg3 peptide-dependent T cell proliferative response of PV patients and healthy individuals in a cross-sectional study.

### Type 2 T cell responses against Dsg3 in PV

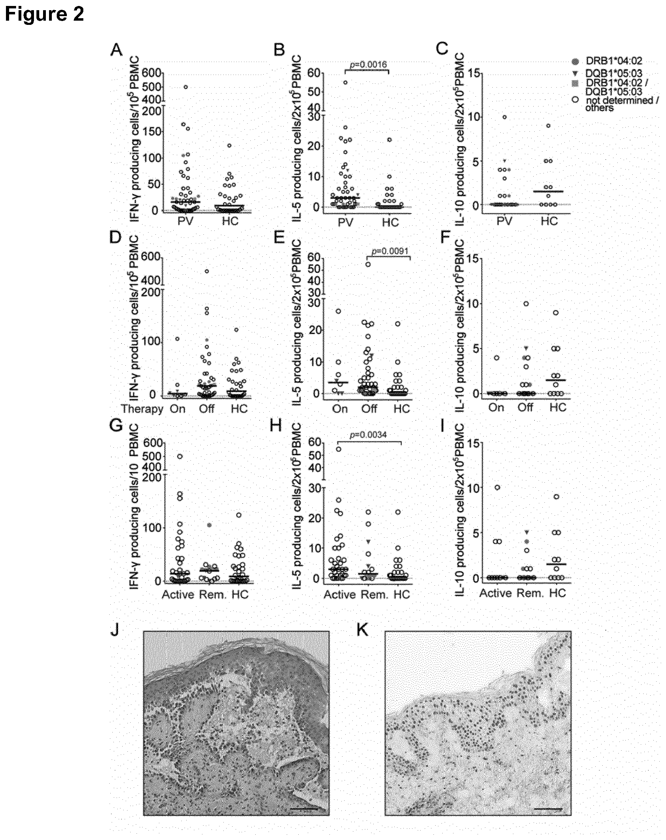

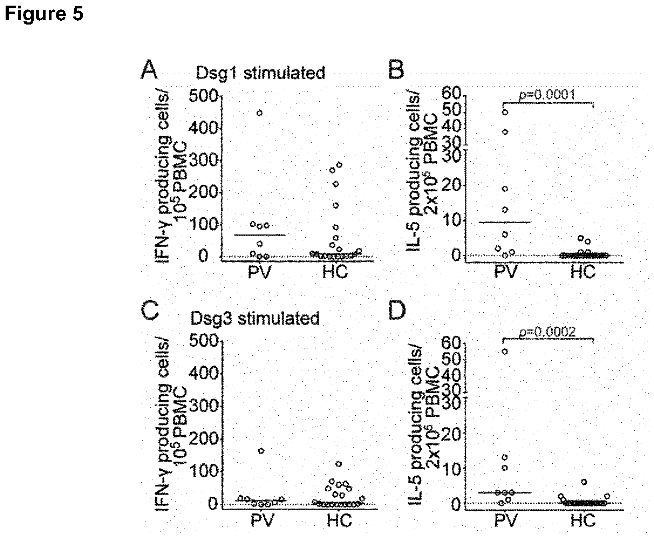

Since they observed a proliferative response against human Dsg3 (i.e. Dsg3₍₁₋₅₆₆₎, SEQ ID NO:9) not only in PV patients but also in healthy carriers of PV-associated HLA class II alleles, the inventors aimed at further characterizing Dsg3-specific T cells by ELISpot assay. As compared to HC, PV patients showed higher numbers of Dsg3-specific IL-5-producing T cells (*p*= 0.0016) by ELISpot analysis, while there was no significant difference in the numbers of IFN-γ- or IL-10-secreting peripheral T cells upon in vitro challenge with Dsg3 (Fig. 2A-C). Moreover, patients with mucocutaneous PV and anti-Dsg3 and anti-Dsg1 serum IgG also showed higher numbers of Dsg1-specific IL-5-producing type 2 T cells compared to HC (Fig. 5).

When PV patients were stratified according to immunosuppressive treatment (Table 1) versus off-therapy (Fig. 2D-F) or active versus remittent disease (Fig. 2G-I), there were no statistically significant differences. Noteworthy, PV patients who were off therapy or in an active phase of the disease showed more IL-5 producing Dsg3-reactive T cells compared to HC (*p*= 0.0091 and *p*= 0.0034, respectively) while no differences were detected for IFN-γ- and IL-10-secreting T cells upon *ex vivo* stimulation with Dsg3. Skin lesions of a representative PV patient with acute disease manifestation prior to treatment showed an intraepidermal infiltrate of eosinophils (Fig. 2J) and a predominantly GATA-3+ type 2 T cell infiltrate (Fig. 2K).

In summary, these results strongly suggest that Dsg3-specific autoreactive T cells from PV patients have a type 2 signature (Th2 cells).

Typically, T cells producing IFN-γ, IL-2 and TNF-α are classified as type 1 T cells (Th1 cells), whereas type 2 T cells (Th2 cells) produce IL-4, IL-5, IL-6, IL-10 and IL-13. The most important function of type2-polarized CD4⁺ T cells is the interaction with B lymphocytes, which takes place via cytokines and cell-derived molecules and leads to the production and release of antibodies by them. This is essential for the humoral immune response and occurs through the typical cytokine pattern of type 2 CD4⁺ T cells (IL-4, IL-5, IL-6, IL-10, IL-13, and iymphotoxin-α).

### Detection of Dsg3 peptide-specific peripheral T cells by HLA-DRB1*04:02 dextramer technology

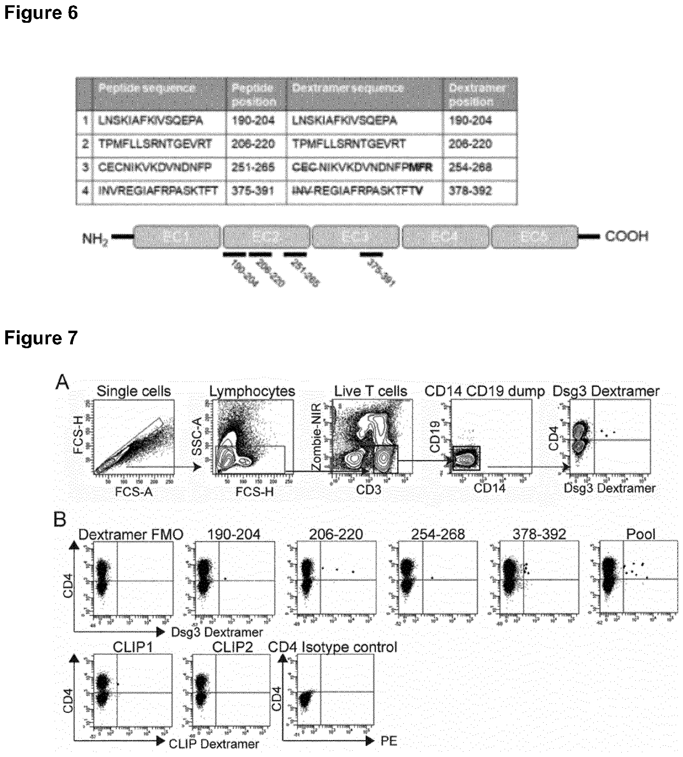

Since the inventors found a pronounced Dsg3-specific type 2 immune response in PV patients, they further explored the epitope specificity of peripheral Dsg3-reactive CD4⁺ T cells. They utilized HLA class II dextramer technology with Dsg3 peptide sequences analogous to the previously identified Dsg3 peptides (see Examples). Immunodominant epitopes of Dsg3, Dsg3₍₁₉₀₋₂₀₄₎, Dsg3₍₂₀₆₋₂₂₀₎, Dsg3₍₂₅₄₋₂₆₈₎, and Dsg3₍₃₇₈₋₃₉₂₎ were coupled to fluorochrome-labeled HLA-DRB1*04:02 dextramers (see Hertl et al., "T cell control in autoimmune bullous skin disorders", J. Clin. Invest. 2006 May;116(5):1159-66. doi: 10.1172/JCI28547.J Clin Invest. 2006. PMID: 16670756; K. Wieber at al., "Detection of autoreactive CD4+ T cells by MHC class II multimers in HLA-linked human autoimmune diseases", J Clin Invest. 2021 May 3;131(9):e148674, doi: 10.1172/JCI148674) and employed for flow cytometric analysis. Dextramers show superior sensitivity compared to tetramers due to enhanced numbers of incorporated HLA class II-peptide-complexes. Two of four sequences of Dsg3 peptides were minimally shifted (Fig. 6). The sequences used are shown in SEQ ID NO: 1 to SEQ ID NO:8. A comparison of the sequences is given in Fig. 6.

For all sequences disclosed in this application, one to three amino acids may be deleted, added and/or substituted by other amino acids, preferably at the ends of the sequences, more preferably only one amino acid.

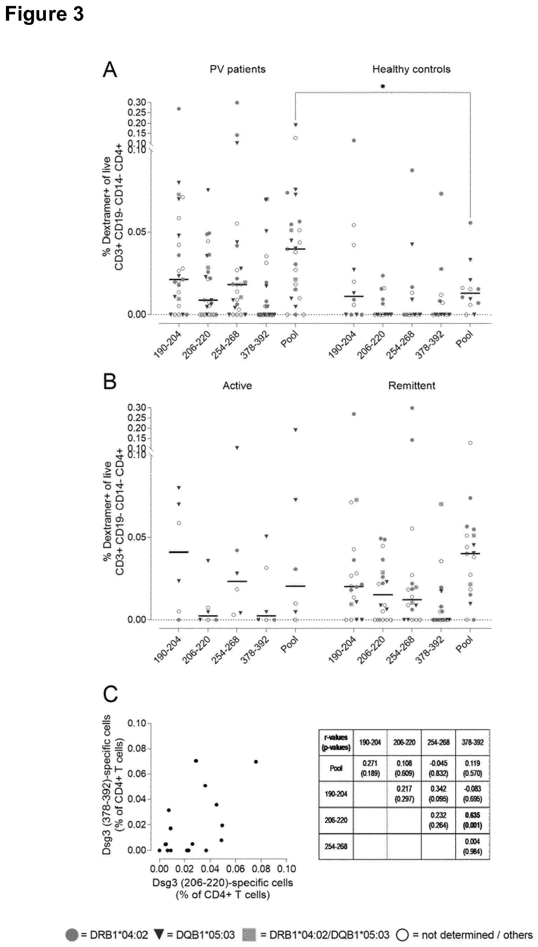

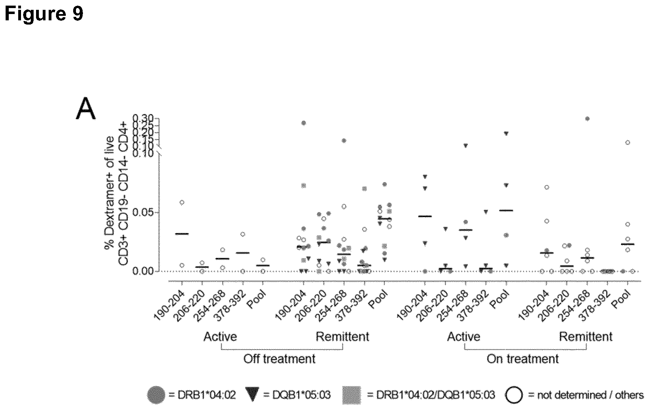

Patient PBMC (peripheral blood mononuclear cells) were stimulated *ex vivo* with recombinant Dsg3 protein, Dsg3₍₁₋₅₆₆₎ (SEQ ID NO:9), which was produced in a bacuovirus expression system, for 7 days and subsequently stained with HLA-DRB1*04:02 dextramers presenting Dsg3 peptides as shown in SEQ ID NO: 1 to SEQ ID NO:8 prior to phenotypical analysis. The class II-associated invariant chain peptide (CLIP) served as control (Fig. 7). HLA-DRB1*04:02 dextramer positive, Dsg3 peptide-reactive CD4⁺ T cells were detected at low frequencies in the majority of PV patients and several HLA-matched HC (Fig. 3A). As expected, the highest numbers of dextramer-positive T cells (>0.3% of gated CD4⁺ T cells) were detected in carriers of HLA-DRB1*04:02 followed by HLA-DQB1*05:03, both in PV patients and healthy donors. Overall, PV patients showed statistically significant higher frequencies of T cells specific for the set of investigated Dsg3 peptides than HC (*p*= 0.0224) (Fig. 3A). Frequencies of Dsg3 peptide-specific cells were similar in active and remittent patients (Fig. 3B) and presumably independent of treatment (Fig. 9). In particular, T cell responses against two Dsg3 epitopes, Dsg3₍₂₀₆₋₂₂₀₎ and Dsg3₍₃₇₈₋₃₉₂₎ were directly correlated (Fig. 3C, *p*= 0.001) while T cell responses against other epitopes of the Dsg3 ectodomain were not.

### Frequencies of Dsg3 peptide-specific peripheral blood T cells and Dsg3-specific IgG serum autoantibodies

The generation of anti-Dsg3 IgG autoantibodies is a critical step in the pathogenesis of PV. Several studies have demonstrated the importance of autoreactive CD4⁺ T cells for autoantibody production in PV in providing essential co-stimulation to autoreactive B cells.

The inventors thus sought to investigate the relationship between Dsg3-specific CD4⁺ T cells, detected by HLA-class-II dextramers, and anti-Dsg3 IgG serum levels. However, no correlation was found between the numbers of T cells specific for any of the immunodominant Dsg3 peptides and anti-Dsg3 or anti-Dsg1 serum IgG (see Table 2 below). Still, the overall number of HLA class II Dsg3 peptide dextramer-positive T cells was higher in the studied PV patients than in the HLA-matched HC (Fig. 3A).

**Table 2: Immunization of HLA-DRB1*04:02-transgenic mice with epitopes residing in the Dsg3 ectodomain leads to the induction of pathogenic anti-Dsg3 IgG antibodies**

| **r-values (p-values)** | Dsg3 | Pool | 190-204 | 206-220 | 254-268 | 378-392 |
|---|---|---|---|---|---|---|
| Anti-Dsg1 | 0.020 (0.931) | 0.385 (0.077) | 0.219 (0.328) | -0.004 (0.986) | 0.205 (0.361) | 0239 (0.283) |
| Anti-Dsg3 | | -0.256 (0250) | -0.204 (0.362) | 0.134 (0.553) | 0.088 (0.696) | -0.086 (0.704) |

Frequencies of Dsg3 peptide reactive T cells of PV patients (n=25) identified in dextramer assays (Fig. 3) were correlated with anti-Dsg3 IgG titers determined by ELISA using Pearson correlation and are expressed by correlations coefficients and p values in Table 2.

### Immunization of HLA-DRB1*04:02-transgenic mice with epitopes residing in the Dsg3 ectodomain leads to the induction of pathogenic anti-Dsg3 IgG antibodies

To further delineate the pathophysiologic relevance of Dsg3 peptide-specific T cells, the inventors took advantage of a previously established HLA-DRB1*04:02-transgenic preclinical mouse model of PV (R. Eming et al., "Pathogenic IgG antibodies against desmoglein 3 in pemphigus vulgaris are regulated by HLA-DRB1*04:02-restricted T cells", J Immunol. 2014 Nov 1;193(9):4391-9. doi: 10.4049/jimmunol.1401081. Epub 2014 Sep 24). Mice were either immunized with recombinant Dsg3 ectodomain (Dsg3₁₋₅₆₆), one of four immunodominant Dsg3 peptides or all the four pooled Dsg3 peptides (Fig. 6) or the pooled peptides wherein slightly shifted peptides of SEQ NO:7 and SEQ ID NO:8 where used in place of SEQ ID NO:3 and SEQ ID NO:4.

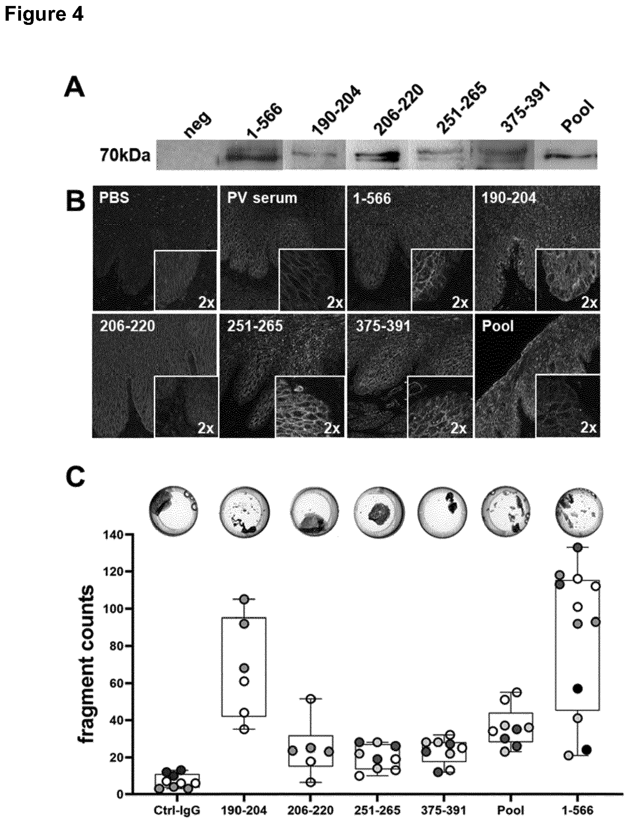

The induction of serum anti-Dsg3 IgG antibodies was evaluated by ELISA, immunoblot analysis with human recombinant Dsg3, and by indirect immunofluorescence microscopy on monkey esophagus. Sera from mice immunized either with the Dsg3 ectodomain (Dsg3₁₋₅₆₆) or with the immunodominant Dsg3 peptide, Dsg3_{(190 204)} of SEQ ID NO:1 showed IgG reactivity with recombinant human Dsg3 and stained the cell surface of epithelial cells of monkey esophagus in a pattern identical to that seen with PV antibodies (Fig. 4A and 4B). Moreover, these immunized mouse sera showed a remarkable loss of cell-cell adhesion of cultured human keratinocytes in the dispase dissociation assay (Fig. 4C). Sera from mice immunized with Dsg3₍₂₀₆₋₂₂₀₎ (SEQ ID NO:2), Dsg3₍₂₅₁₋₂₆₅₎ (SEQ ID NO:3) and Dsg3₍₃₇₅₋₃₉₁₎ (SEQ ID NO: 4) also induced loss of keratinocyte adhesion, although to a lesser extent (Fig. 4C). Of note, sera from the mice immunized with the later three immunodominant Dsg3 epitopes all reacted with recombinant human Dsg3 (Fig. 4A) but showed different staining patterns with epithelial cells of monkey esophagus (Fig. 4B). While sera from the mice immunized with Dsg3₍₂₅₁₋₂₆₅₎ and Dsg3₍₃₇₅₋₃₉₁₎ (SEQ ID NO: 3 and SEQ ID NO:4, respectively) showed the characteristic PV-like cell surface staining pattern on monkey esophagus epithelium, sera from the Dsg3₍₂₀₆₋₂₂₀₎-immunized mice (SEQ ID NO: 2) showed a cytoplasmatic staining pattern (Fig. 4B).

These findings suggest a polyclonal autoreactive T cell response in PV which is associated with the induction of IgG antibodies of different epitope specificities, binding affinities and pathogenicity. This contention is supported by the finding that T cell responses against two distinct Dsg3 peptides, Dsg3₍₂₀₆₋₂₂₀₎ (SEQ ID NO: 2) and Dsg3₍₃₇₈₋₃₉₂₎ (SEQ ID NO: 8), were found to be correlated to each other (Fig. 3C).

The inventors aimed at identifying and further characterizing the Dsg3-specific T cellular immune response in PV with regard to cytokine profile and epitope specificity. Their results show a pronounced Dsg3-specific type 2 peripheral T cell response against the major autoantigen of PV, Dsg3 (extracellular part shown in SEQ ID NO:9). Utilizing HLA class II Dsg3 peptide dextramer technology, autoreactive T cells specific for distinct immunodominant Dsg3-peptides were detected in the peripheral blood of PV patients and of HLA class II-matched HC. Peripheral T cells specific for a specific set of epitopes in the Dsg3 ectodomain were found at higher frequencies in PV patients than in healthy individuals. Furthermore, in a preclinical HLA class-transgenic mouse model of PV, the same set of Dsg3 peptides induced pathogenic anti-human Dsg3 IgG antibodies which conferred loss of epidermal keratinocyte adhesion *in vitro.*

These findings strongly suggest that distinct Dsg3 epitope-specific T cells are critical in the pathogenesis of PV and may serve as therapeutic targets.

The inventors here provide additional evidence that PV is a type 2 T cell mediated autoimmune disorder since the patients' sera frequently contain auto-antibodies of the IgG4 and IgE subclasses against the target antigen Dsg3. Several Th2-cytokines, including IL-4, IL-5 or IL-13 have been found to be elevated in PV sera. Using ELISpot assay, the inventors observed a pronounced peripheral blood type 2 immune response in PV patients upon stimulation with Dsg3 as compared to HC (Fig. 2). Of note, healthy individuals showed Dsg3-specific type 1 immune response with IFN-γ-secreting T cells comparable to PV patients, suggesting a less disease-promoting function of these cells as described in previous studies. IL-10-producing T cells tended to be increased in HC but did not reach statistical significance, presumably due to the limited number of PV patients included in the analysis. However, previous studies already demonstrated that Dsg3-autoreactive, IL-10-producing T regulatory (Treg) cells are present to a greater extent in healthy individuals than in PV patients. Moreover, peripheral blood CD4⁺CD25^{high} Treg cells were previously found to be markedly decreased in PV.

Collectively, the present ELISpot analysis extends previous studies demonstrating that autoreactive Th2 cells are preferentially detected in PV and further supports the concept that pemphigus is an autoimmune disorder of the skin, which is orchestrated by pathogenic type 2 T cells. This contention is also supported by a known type 2 T cell infiltrate in PV skin lesions associated with eosinophils and the detection of anti-Dsg3 IgG4 and IgE in the sera of PV patients.

In addition to the observed autoreactive type 2 T cell response, Th17 cells may be also involved in the immune pathogenesis of PV suggested by elevated serum IL-17 levels. Studies from other laboratories showed that peripheral Th17 cells (defined by CXCR3 and CCR6 surface expression) did not differ between PV patients and HC. In a recent study of the inventors' laboratory, IL-17-secreting CXCR3 - CCR6 + T follicular helper (Tfh)17 cells were significantly increased in pemphigus patients with an acute proinflammatory state of disease and were able to induce the production of Dsg3-specific IgG after co-incubation with memory B cells *in vitro* (J. Holstein et al. "Immunophenotyping in pemphigus reveals a Th17/Tfh17-dominated immune response promoting desmoglein1/3-specific autoantibody production", J Allergy Clin Immunol 2020; 10.1016/j.jaci.2020.11.008).

The inventors' present results, further, extend previous findings from their group and others that peripheral blood autoreactive type 2 T cells recognize a limited set of distinct epitopes of the Dsg3 ectodomain in association with two PV-associated HLA class II alleles, namely HLADRB1*04:02 and HLA-DQB1*05:03.

In contrast, healthy carriers of the PV-associated HLA class II alleles preferentially carry Dsg3-reactive Th1 cells, further underlining the critical role of HLA class II-restricted T cell recognition of autoantigenic peptides in PV.

In contrast to a previous study with open HLA-DRB1*04:02 tetramers loaded with Dsg3 peptides (C. M. Veldman et al., 2004; a.a.O), the inventors now found a higher sensitivity in detecting Dsg3 peptide-specific T cells utilizing HLA-DRB1*04:02 dextramers loaded with the identical or similar Dsg3 peptides. Frequencies of dextramer positive T cells were in the range of 0.001-0.3 % of peripheral blood CD4⁺ T cells. The latter rates are comparable to those of a previous study, which estimated a precursor frequency of approximately 3-10 Dsg3-specific T cells per 10⁵ peripheral blood mononuclear cells (PBMC) (C.M. Veldman et al., 2004, a.a.O.).

To improve the detection of low frequency autoreactive precursor T cells, PBMC of the studied patients were first enriched for autoreactive T cells by *ex vivo* stimulation with Dsg3 followed by an optimized staining protocol that includes addition of the protein kinase inhibitor dasatinib and anti-phycorythrin (PE)-antibody, preventing TCR downregulation and stabilizing of TCR-dextramer interaction, respectively.

These findings are in line with the detection of low frequency autoreactive precursor T cells in a variety of human HLA class II-associated autoimmune diseases including myasthenia gravis, multiple sclerosis, gluten-sensitive enteropathy utilizing MHC class II-peptide tetramers as reported previously (K. Wieber et al., "Detection of autoreactive CD4+ T cells by MHC class II multimers in HLA-linked human autoimmune diseases", J Clin Invest 2021; 131, 10.1172/JCI148674).

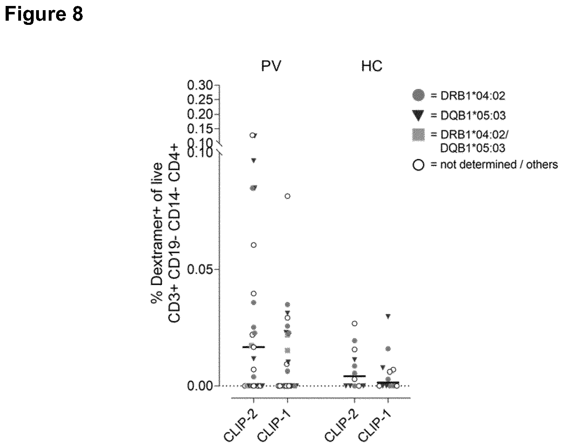

The inventors also used HLA-DRB1*04:02 dextramers with CLIP peptides as control (Fig. 8). The frequencies of HLA-DRB1*04:02 CLIP peptide dextramer⁺ peripheral T cells did not differ significantly between PV patients and HC (Fig. 8) but were lower than described in the literature as PBMC from PV patients and HC were propagated *ex vivo* with human Dsg3 to enrich the frequency of autoreactive T cells prior to dextramer analysis.

Autoreactive T cells were preferentially detected in PV patients with the disease-associated HLA class II allele, HLA-DRB1*04:02. PV patients expressing another PV-associated HLA class II allele, HLA-DQB1*05:03, which possesses peptide binding motifs analogous to HLA-DRB1*04:02 also carried T cells specific for these Dsg3 peptides as shown by dextramer technology (Fig. 3A).

The pathogenic relevance of T cells specific for distinct Dsg3 peptides of this invention has been confirmed in an HLA class II transgenic mouse model established by the inventors' group. In this model, immunization of the mice with human Dsg3 leads to induction of Dsg3-specific T cells which recognize the same epitopes of Dsg3 in association with HLA-DRB1*04:02 which had been previously identified in PV patients. Thus, the inventors evaluated the pathogenicity of Dsg3-specific autoantibodies induced after immunizing HLA-transgenic mice with the set of immunodominant Dsg3-pepides (Fig. 4). As the frequencies of T cell responses against two distinct Dsg3 epitopes were directly correlated to each other (Fig. 3C), it was assumed that T cells of different Dsg3 peptide specificities were able to induce pathogenic anti-Dsg3 IgG responses in concert. In fact, induction of pathogenic antibodies was observed upon immunization of the mice with several Dsg3 peptides, i.e. Dsg3₍₁₉₀₋₂₀₄₎, Dsg3₍₂₀₆₋₂₂₀₎, Dsg3₍₂₅₁₋₂₆₅₎, and Dsg3₍₃₇₅₋₃₉₁₎. (SEQ ID NO: 1, SEQ ID NO: 2, SEQ ID NO:3 and SEQ ID NO:4). Of note, serum antibodies induced by Dsg3₍₁₉₀₋₂₀₄₎ peptide (SEQ ID NO: 1) immunization showed the highest pathogenic capacity as compared to recombinant human Dsg3. These findings are in line with previous observations made by the Amagai group in an autologous mouse PV mouse model directed against murine Dsg3. Specifically, they identified T cell responses against the NH₂-terminal subdomain of murine Dsg3 which provided help to B cells to produce pathogenic anti-Dsg3 IgG (H. Takahashi et al., "Novel System Evaluating In Vivo Pathogenicity of Desmoglein 3-Reactive T Cell Clones Using Murine Pemphigus Vulgaris", J Immunol 2008; 181; 1526-1535; 10.4049/jimmunol.181.2.1526). Of note, they also detected T cell responses against other epitopes of Dsg3 which did not induce pathogenic or only less pathogenic anti-Dsg3 IgG. Along this line, Kawasaki et al. showed that anti-Dsg3 IgG antibodies which were secreted by some B cell hybridomas generated from mice immunized with murine Dsg3 were less pathogenic (H. Kawasaki et al., "Synergistic pathogenic effects of combined mouse monoclonal anti-desmoglein 3 IgG antibodies on pemphigus vulgaris blister formation", J Invest Dermatol 2006; 126:2621-30.; 10.1038/sj.jid.5700450). However, these anti-Dsg3 IgG antibodies were shown to act synergistically, leading to overt blister formation *in vivo* (passive transfer into mice) and *in vitro* (dissociation assay). These findings were recently confirmed in PV patients by Cho et al. (A. Cho et al., "Single-Cell Analysis Suggests that Ongoing Affinity Maturation Drives the Emergence of Pemphigus Vulgaris Autoimmune Disease", Cell Rep 2019; 28:909-922.e6.; 10.1016/j.celrep.2019.06.066).

In conclusion, the inventors' findings are in line with the current concept that the Dsg3-driven T and B cell response in pemphigus is rather polyclonal and heterogeneous despite a predominance of autoreactive T cells specific for distinct Dsg3 epitopes.

However, the determination of specific T-cells and their cytokine profile can nevertheless been used in the claimed method for diagnosis and determination of disease activity, as described above.

### Description of Figures

Fig. 1 shows the proliferative response of peripheral blood mononuclear cells (PBMC) from PV patients and healthy carriers of PV-associated HLA class II alleles to Dsg3 protein and T cell epitopes of Dsg3. PBMC were stimulated *in vitro* with the extracellular domain of Dsg3 (Dsg3₍₁₋₅₆₆₎) (SEQ ID NO:9), expanded with exogenous IL-2 and were subsequently restimulated with Dsg3₍₁₋₅₆₆₎ or Dsg3 peptides (Dsg3₍₁₉₀₋₂₀₄₎ SEQ ID NO.1 and 5), Dsg3₍₂₀₆₋₂₂₀₎ (SEQ ID NO: 2 and 6), Dsg3₍₂₅₁₋₂₆₅₎ (SEQ ID NO:3), Dsg3₍₃₇₅₋₃₉₁₎ (SEQ ID NO:4)), respectively. The proliferative T cell response was determined by the uptake of ³H-thymidine. Shown are the ratios of stimulated and unstimulated cells (stimulation index) for PV patients (n=9-17) and HC (n=10) (Fig. **1A**). T cell responses of the PV patients were stratified according to immunosuppressive therapy for untreated patients (n=7) or patients on therapy (n=2-10) (Fig. **1B**) and active disease (n=3-6) versus remission (n=6-11) (Fig. **1C**). Patients and controls carrying the PV-associated HLA class II alleles, HLA-DRB1*04:02 or HLA-DQB1*05:03, are indicated as circles and triangles, respectively, while empty circles show PV patients and HC with unknown or unrelated HLA class II alleles.

Fig. 2 shows the cytokine profile of peripheral blood T cells from PV patients and healthy carriers of PV-associated HLA class II alleles. The cytokine secretion of PBMC stimulated *ex vivo* with Dsg3₍₁₋₅₆₆₎ (SEQ ID NO:9) was studied by ELISpot assay. Shown are numbers of IFN-γ-, IL-5- and IL-10-secreting cells, respectively, from PV patients (n=26-49) and HC (n=10-30) (Fig. **2A-C**). PV patients were also stratified according to immunosuppressive therapy versus off-therapy (Fig. **2D-F**) and active versus remittent disease (Fig. **2G-I**). Patients and healthy carriers of HLA-DRB1*04:02 and HLA-DQB1*05:03 are shown as circles and triangles, respectively, while empty circles represent patients and HC with unknown or unrelated HLA class II alleles. Where present, a significant statistical difference is indicated with *p* value as determined by two-tailed nonparametric Mann-Whitney-U-Test.

Histopathological examination of a skin lesion shows epidermal spongiosis with numerous eosinophils (Fig. **2J**), and a GATA-3+ T cell infiltrate by immunohistochemistry (Fig. **2K**).

Fig. 3 depicts the detection of Dsg3-peptide-specific CD4⁺ T cells in PV patients and healthy carriers of PV-associated HLA class II alleles using HLA-DRB1*04:02-Dsg3 peptide dextramers. PBMC were co-cultured with the extracellular domain of Dsg3 (Dsg3₍₁₋₅₆₆₎) (SEQ ID NO:9) and subsequently stained with HLA-DRB1*04:02 dextramers coupled to either Dsg3₍₁₉₀₋₂₀₄₎ (SEQ ID NO:5), Dsg3₍₂₀₆₋₂₂₀₎ (SEQ ID NO: 6), Dsg3₍₂₅₄₋₂₆₈₎ (SEQ ID NO:7), and Dsg3₍₃₇₈₋₃₉₂₎ (SEQ ID NO: 8) or a pool of all 4 dextramers-specificities. Shown are the frequencies of Dsg3 peptide-specific CD4⁺ T cells from PV patients (n=25) and HC (n=12) (Fig. **3A**). T cell data from the PV patients were also stratified according to disease activity (active: n=6, remittent: n=18) (Fig. **3B**). Lines indicate medians. Where present, a significant statistical difference is indicated with *p* value as determined by two-tailed nonparametric Mann-Whitney-U-Test. Shown are patients and healthy carriers of HLA-DRB1*04:02 (circles), HLA-DQB1*05:03 (triangles) and both HLA class II alleles (squares), respectively, while empty circles represent patients and controls with unknown or differing HLA class II alleles. Correlations between Dsg3 peptide-specific T cells from PV patients (n=25) were analysed using Pearson correlation and are expressed by correlations coefficients and p values in Table 2. Additionally, frequencies of Dsg3 peptide-reactive cells against epitopes 206-220 (SEQ ID NO: 2) and 378-392 (SEQ ID NO: 8) are plotted (Fig. **3C**).

Fig. 4 shows that the immunization of HLA-DRB1*04:02-transgenic mice with immunodominant Dsg3 T cell epitopes leads to the formation of pathogenic IgG antibodies. To further elucidate the relative role of T cells specific for defined immunodominant epitopes of Dsg3, HLA-DRB1*04:02-transgenic mice were immunized with single or pooled immunodominant Dsg3 peptides (Dsg3₍₁₉₀₋₂₀₄₎, Dsg3₍₂₀₆₋₂₂₀₎, Dsg3₍₂₅₁₋₂₆₅₎, and Dsg3₍₃₇₅₋₃₉₁₎, SEQ ID NO: 1, 2, 3 and 4) or the entire Dsg3 ectodomain of Dsg3 (Dsg3₍₁₋₅₆₆₎) (SEQ ID NO:9). The evolving T cell-dependent anti-Dsg3 IgG response was visualized by immunoblot analysis (Fig. **4A**) and indirect immunofluorescence staining on monkey esophagus epithelial cells (Fig. **4B**). Pathogenicity of serum anti-Dsg3 antibodies was evaluated by means of keratinocyte dissociation assay (Fig. **4C**). Representative illustrations of resulting fragments are shown and cumulative results of 3 independent experiments with standard deviation are shown. Pooled mouse serum was used as negative control IgG and the recombinant entire Dsg 3 ectodomain (1-566) (SEQ ID NO:9) as a positive control, respectively. Significant statistical differences are indicated as determined by Kruskal-Wallis one-way ANOVA test.

Fig. 5 shows the cytokine profile of peripheral blood T cells from PV patients and healthy carriers of PV-associated HLA class II alleles. The cytokine secretion of PBMC stimulated *ex vivo* with Dsg1₍₁₋₅₆₅₎ (SEQ ID NO:10), Fig. **5A-B**) and Dsg3₍₁₋₅₆₆₎ (SEQ ID NO:9, Fig. **5C-D**) was studied by ELISpot assay. Shown are numbers of IFN-γ-(Fig. **5A****,C**) and IL-5- (Fig. **5B****,D**) secreting cells, respectively, from PV patients (n=8) and HC (n=20). All the studied PV patients had anti-Dsg3 IgG and/or anti-Dsg1 IgG. Where present, a significant statistical difference is indicated with p value as determined by two-tailed nonparametric Mann-Whitney-U-Test.

Fig. 6 shows the amino acid sequences of soluble immunodominant desmoglein (Dsg) peptide sequences and HLA class II-Dsg3 peptide dextramers. The sequences are reproduced in SEQ. ID Nos: 1 to 8, respectively.

Previously identified immunodominant peptides of Dsg3 were synthesized and used for proliferation assays (Fig. 1) and immunization of mice (Fig. 4) (SEQ ID NO: 1 to 4). Immunodominant peptide sequences aa 251-265 and aa 375-391 were minimally shifted for coupling to dextramers (Fig. 3). Graphic adapted from Veldman et al., 2004 (a.a.O.). That is, for stable peptide binding to HLA-DRB1*04:02 dextramers immunodominant peptides needed to be minimally modified, i.e. amino acids were omitted (crossed out) or added (bold), resulting in partially altered amino acid sequences in HLA class II-Dsg3 peptide dextramer assays (Fig. 3) (SEQ ID NO:7 and 8).

Fig. 7 depicts the gating strategy to identify HLA-DRB1*04:02-Dsg3 peptide dextramer⁺ T cells. (Fig. **7A**): Dextramer positive CD4⁺ T cells were identified as CD3⁺ CD14⁻ CD19⁻CD4⁺ dextramer⁺ live lymphocytes. (Fig. **7B**): Representative FACS plots showing CD4⁺ T cells stained with dextramers coupled to the indicated Dsg3 peptide sequence, the combination of a dextramers (pool), the dextramer control with the class II-associated invariant chain peptide (CLIP) and the CD4 isotype control.

Fig. 8 shows the detection of CLIP-specific CD4⁺ T cells in PV patients and HC by HLA-DRB1*04:02 dextramers. The class II-associated invariant chain peptide (CLIP) served as control in our dextramer assays. Frequencies of CLIP-reactive CD4⁺ T cells are shown for PV patients (left, n=25) and HC (right, n=12). CLIP-2 represents staining control for single peptides, while CLIP-1 serves as control for staining for the pool of the four investigated peptides. Lines indicate medians. Shown are patients and healthy carriers of HLA-DRB1*04:02 (circles), HLA-DQB1*05:03 (triangle) and both HLA class II alleles (squares), respectively, while empty circles represent patients and controls with unknown or unrelated HLA class II alleles.

Fig. 9 shows frequencies of Dsg3 peptide-specific CD4⁺ T cells in PV patients respective to disease activity and treatment. To investigate a possible impact of disease activity and treatment, data were stratified accordingly. Active and untreated PV (n=2), remittent and untreated PV (n=12), active and treated (n=4), remittent and treated (n=6). Lines indicate medians. Shown are patients and healthy carriers of HLA-DRB1*04:02 (circles), HLA-DQB1*05:03 (triangle) and both HLA class II alleles (squares), respectively, while empty circles represent patients and controls with unknown or unrelated HLA class II alleles.

### Examples

### Patients and healthy controls

A total of 52 PV patients and 41 HC were enrolled. Overall, 28 PV patients had active disease (acute: 21, chronic: 7) and 23 were in complete clinical remission. The characteristics of patients are shown in Table 1 below.

Diagnosis of PV was confirmed by the presence of fragile blisters and/or erosions of the mucous membranes and skin, epidermal cells surface deposits of IgG and/or C3 by direct immunofluorescence (DIF) and anti-Dsg3 serum IgG by ELISA (M. Hertl et al., "Pemphigus. S2 Guideline for diagnosis and treatment - guided by the European Dermatology Forum (EDF) in cooperation with the European Academy of Dermatology and Venereology (EADV)", J Eur Acad Dermatol Venereol 2015; 29:405-14; 10.111/jdv.12772). All participants received oral and written information and signed a written consent prior to inclusion in the study. All study protocols were reviewed and approved by the Ethics Committee of the Medical Faculty of Philipps-University, Marburg, according to the Declaration of Helsinki Principles (AZ 20/14).

**Table 1: Clinical and immune serological characteristics and HLA class II haplotypes of studied PV patients**

| **Pat.** | **Disease activity** | **anti-Dsg1 (RE/ml)** | **anti-Dsg3 (RE/ml)** | **Immunosuppressive treatment (per day)** | **HLA-DQB1*** | **HLA-DRB1*** |
|---|---|---|---|---|---|---|
| 1. | acute | 0 | 1100 | none | n.a. | n.a. |
| 2. | acute | n.a. | n.a. | none | n.a. | n.a. |
| 3. | acute | n.a. | n.a. | none | n.a. | n.a. |
| 4. | acute | 0 | 1260 | none | n.a. | n.a. |
| 5. | acute | n.a. | n.a. | none | n.a. | n.a. |
| 6. | acute | 65 | 1060 | none | n.a. | n.a. |
| 7. | acute | 290 | 1670 | none | n.a. | n.a. |
| 8. | acute | 0 | 95 | none | n.a. | n.a. |
| 9. | acute | 0 | 176 | none | n.a. | n.a. |
| 10. | acute | 630 | 990 | none | n.a. | n.a. |
| 11. | acute | 73 | 1160 | none | n.a. | n.a. |
| 12. | acute | 360 | 159 | none | n.a. | n.a. |
| 13. | acute | 0 | 1450 | none | n.a. | n.a. |
| 14. | acute | 0 | 39 | none | n.a. | n.a. |
| 15. | acute | 22 | 880 | none | n.a. | n.a. |
| 16. | acute | 0 | 30 | none | n.a. | n.a. |
| 17. | acute | 73 | 405 | none | n.a. | n.a. |
| 18. | remittent | 1 | 21 | none | **05:03** | 13:01 |
| 19. | chronic | 3 | 210 | Prednisone 5 mg | **05:03** | 14:54 |
| 20. | chronic | 0 | 166 | none | 01:02 | 07:01 |
| 21. | remittent | 0 | 200 | none | 30:20 | **04:02** |
| 22. | chronic | 0 | 72 | Prednisone 30 mg | 03:01 | 14:54 |
| 23. | remittent | 0 | 191 | none | 03:01 | 13:05 |
| | | | | | 04:02 | 08:04 |
| 24. | chronic | 2 | 35 | Azathioprine 150 mg | **05:03** | 13:01 |
| | | | | Prednisone 5 mg | 06:03 | 14:01 |
| 25. | remittent | 7 | 0 | none | 05:01 | 01:02 |
| | | | | | 05:02 | 14:01 |
| 26. | remittent | 0 | 78 | none | **05:03** | 13:01 |
| | | | | | 06:03 | 14:01 |
| 27. | remittent | 0 | 0 | none | 06:03 | **04:02** |
| | | | | | 03:02 | 03:01 |
| 28. | remittent | 10 | 157 | none | **05:03** | **04:02** |
| | | | | | 03:02 | 14:01 |
| 29. | remittent | 20 | 270 | Prednisone 5 mg | 03:01 | **04:02** |
| | | | | | 03:02 | 11:01 |
| 30. | remittent | 0 | 2 | none | 03:01 | **04:02** |
| | | | | | 03:02 | 11:01 |
| 31. | remittent | 0 | 32 | none | 03:01 | **04:02** |
| | | | | | 03:02 | 11:04 |
| 32. | chronic | 0 | 20 | none | 03:01 | 14:54 |
| | | | | | **05:03** | 08:04 |
| 33. | remittent | 5 | 4 | none | **04:02** | **04:02** |
| | | | | | 03:02 | 08:01 |
| 34. | remittent | 2 | 3 | none | n.a. | n.a. |
| 35. | remittent | 3 | 683 | none | **05:03** | 03:-- |
| | | | | | 02:-- | 14:-- |
| 36. | remittent | 5 | 2 | none | 03:02 | 14:-- |
| | | | | | **05:03** | **04:02** |
| 37. | acute | 4 | 94 | Prednisone 20 mg | 01:01 | 05:01 |
| | | | | Azathioprine 100 mg | 11:04 | 03:01 |
| 38. | chronic | 92 | 101 | none | n.a. | n.a. |
| 39. | acute | 117 | 20 | Azathioprine 100 mg | **05:03** | 13:01 |
| | | | | | 06:03 | 14:04 |
| 40. | chronic | 0 | 0 | Mycophenolate mofetil 2 g | 03:01 | **04:02** |
| | | | | | 03:02 | 11:04 |
| 41. | acute | 186 | 178 | Prednisone 25 mg | **05:03** | 13:01 |
| | | | | | 06:03 | 14:54 |
| 42. | remittent | 7 | 153 | Prednisone 7.5 mg | n.a. | n.a. |
| 43. | remittent | 3 | 334 | none | 03:02 | 13:05 |
| | | | | | 08:04 | **04:02** |
| 44. | remittent | n.a. | n.a. | Rituximab 500 mg | n.a. | n.a. |
| | | | | Prednisone 25 mg | | |
| 45. | remittent | 2 | 39 | Prednisone 2 mg | n.a. | n.a. |
| | | | | Methotrexate 15 mg per week | | |
| 46. | remittent | n.a. | n.a. | none | n.a. | n.a. |
| 47. | remittent | 9 | 33 | Prednisone 4 mg | n.a. | n.a. |
| | | | | Mycophenolate mofetil 1.5 g | | |
| 48. | remittent | 4 | 66 | Prednisone 5mg | n.a. | n.a. |
| | | | | Ciclosporine 150 mg | | |
| 49. | acute | 382 | 177 | none | **05:03** | 01:01 |
| | | | | | 05:01 | 14:54 |
| 50. | remittent | 245 | 0 | Prednisone 5 mg | n.a. | n.a. |
| | | | | Mycophenolate mofetil 2 g | | |
| | | | | Dapsone 50 mg | | |
| 51. | remittent | 9 | 643 | none | **05:03** | 14:01 |
| | | | | | 02:02 | 07:01 |
| 52. | n.a. | n.a. | n.a. | n.a. | 03:02 | 04:01 |
| | | | | | **05:03** | 14:54 |

| | | | | | | |
|---|---|---|---|---|---|---|
| Acute: ≤ 6 week duration; chronic: > 6 week duration; remittent: no blisters/erosions of mucosa and/or skin, both summarized as "active" disease; n.a.: not analyzed RE/ml: relative Einheiten (relative units)/ml HLA-DQB1: major histocompatibilty complex, class II, DQ beta 1, is a human gene and also denotes the genetic locus that contains this gene. The protein encoded by this gene is one of two proteins that are required to form the DQ heterodimer, a cell surface receptor essential to the function of the immune system. The DQ heterodimer consists of an alpha (HQA) and a beta chain (HQB), both anchored in the membrane. It plays a central role in the immune system by presenting peptides derived from extracellular proteins. HLA-DRB1: class II histocompatibility antigen, DRB1 beta chain is a protein that in humans is encoded by the HLA-DRB1 gene. DRB1 encodes the most prevalent beta subunit of HLA-DR. The protein encoded by this gene belongs to the HLA class II beta chain paralogues. The class II molecule is a heterodimer consisting of an alpha (DRA) and a beta chain (DRB), both anchored in the membrane. It plays a central role in the immune system by presenting peptides derived from extracellular proteins to T helper cells. Class II molecules are constitutively expressed in professional antigen-presenting cells (APC: B lymphocytes, dendritic cells, macrophages), and could be induced in non-professional APCs. HLA: human leukocyte antigen system or complex is a complex of genes on chromosome 6 in humans which encode cell-surface proteins responsible of the immune system. | | | | | | |

### Next generation sequencing HLA typing

Allelic resolution HLA typing was performed by next generation sequencing on the illumina platform (MiSeqDx, Illumina, San Diego, CA, USA) employing single loci amplification strategy (GenDx, Utrecht, The Netherlands).

### PBMC Isolation

Peripheral blood from patients and controls was collected in citrate-phosphate-dextrose-adenine (CPDA) anticoagulant tubes (S-Monovette; Sarstedt, Nürnberg, Germany). Peripheral blood mononuclear cells (PBMC) were isolated by density-gradient centrifugation using Lymphocyte Separation Medium (Capricorn, Ebsdorfergrund, Germany) and were cryopreserved in liquid nitrogen at -196°C for later use.

### ELISpot assay

ELISpot assay was performed as previously described (T. Schmidt et al., "TH1/TH17 cell recognition of desmoglein 3 and bullous pemphigoid antigen 180 in patients with lichen planus", J Allergy Clin Immunol 2018; 142:669-672.e7; 10.1016/j.jaci.2018.02.044; C. Möbs, T. Schmidt; "Research Techniques Made Simple: Monitoring of T-Cell Subsets using the ELISPOT Assay", J Invest Dermatol 2016; 136:e55-e59; 10.10106/j.jid.2016.04.009) using human IFN-γ, IL-5 or IL-10 ELISpot Sets (Becton Dickinson, Franklin Lakes, NJ, USA).

Briefly, cryopreserved PBMC were thawed at 37 °C and 10⁶ cells/mL for each stimulatory condition were cultured in RPMI-1640 (Capricorn, Ebsdorfergrund, Germany) supplemented with 10 % pooled human serum, 100 U/mL penicillin, 100 µg/mL streptomycin and 2 mmol/L L-glutamine (Capricorn, Ebsdorfergrund, Germany). PBMC were stimulated with 10 µg/mL of recombinant Dsg3₍₁₋₅₆₆₎ produced in a baculovirus expression vector system as previously described (R. Müller et al., "IgG reactivity against non-conformational NH-terminal epitopes of the desmoglein 3 ectodomain relates to clinical activity and phenotype of pemphigus vulgaris", Exp Dermatol 2006; 15; 606-14; 10.1111/j.1600-0625.2006.00451.x). After 48 hours, IL-2 (10 U/mL; Roche, Mannheim, Germany) and IL-7 (10 ng/mL; Miltenyi Biotech, Bergisch Gladbach, Germany) were added to all wells for *in vitro* expansion of autoreactive T cells. On day 7, PBMC were re-stimulated with 10 µg/mL Dsg3₍₁₋₅₆₆₎ and seeded at 2×10⁵ (IL-5; IL-10) or 1×10⁵ (IFNγ) per well on ELISpot plates coated with the respective antibody. The cells were incubated for 20 hours at 37 °C in a humidified atmosphere containing 5 % CO₂. IFNγ-, IL-5- and IL-10-positive spots were detected according to the manufacturers' instructions. Spots were counted automatically with an ELISpot plate reader (A.EL.VIS, Hannover, Germany). For data analysis, mean spots of the non-stimulated controls (mean) were subtracted from the mean spots of the cultures with antigen (all in duplicate).

### ³H Thymidine incorporation assay

Cryopreserved PBMC were thawed at 37 °C and cells were cultured in RPMI-1640 (Capricorn, Ebsdorfergrund, Germany) supplemented with 10 % pooled human serum, 100 U/mL penicillin, 100 µg/mL streptomycin and 2 mmol/L L-glutamine (Capricorn, Ebsdorfergrund, Germany) in 24-well plates at 2×10⁶ cells per well in the presence of 10 µg/ml recombinant Dsg3₍₁₋₅₆₆₎ protein. After 5 days, IL-2 (10 U/mL; Roche, Mannheim, Germany) was added for *in vitro* expansion of autoreactive T cells. T cells were cultured for a total of 12-14 days until T cell proliferation decreased. Subsequently, 0.25×10⁶ resting T cells were co-cultured with 0.75×10⁶ autologous irradiated (2 × 31 G) PBMC and stimulated with Dsg3 protein, 15-mer Dsg3 peptides (Prolmmune, Oxford, UK) or PHA as positive control in 96-well round bottom plates. Triplicates or Quadruplicates were used for each assay. After 4 days of incubation, ³H-thymidine was added to the T cell cultures (0.5 µCi / well) for 18 hours. Cells were subsequently transferred onto 96-UniFilter plates (PerkinElmer LAS, Rodgau, Germany) and radioactive events (counts per minute; cpm) were determined for each well using a scintillation beta-counter (TopCount NXT, PerkinElmer LAS, Rodgau, Germany).

### HLA class II dextramer assay

Cryopreserved PBMC were thawed at 37 °C and cells were seeded in a 24-well plate (Thermo Scientific, Denmark) at 2 × 10⁶ per well with addition of 10 µg/mL recombinant Dsg3₍₁₋₅₆₆₎ (SEQ ID NO:9) which was produced in a baculovirus expression system, for antigen-specific stimulation. After 48 hours, IL-2 (10 U/mL; Roche, Mannheim, Germany) was added to all the wells for *in vitro* expansion of activated autoreactive T cells. On day 7, the cultured T cells were harvested and subsequently used for flow cytometric analysis. Cells were washed twice with PBS+5 % FCS and incubated with 100 nM Dasatinib (Axon Medchem, Germany) for 30 min at 37 °C. Phycoerythrin (PE)-labeled MHC-II-Dextramers presenting Dsg3 peptides as well as the CLIP peptide (Immudex, Denmark) were added for 30 min at room temperature in the dark. Cells were washed twice with PBS+5 % FCS and anti-PE antibody (PE001, BioLegend, San Diego, USA) was added at the concentration of 10 µg/mL for 20 min at 4 °C as previously described (G. Dolton et al., "Optimized Peptide MHC Multimer Protocols for Detection and Isolation of autoimmune T-cells", Front Immunol 2018; 9; 1378; 10.3389/fimmu.2018.01378). Cells were washed with PBS and subsequently incubated for 20 min at 4 °C in the dark with the following fluorochrome-labeled antibodies: anti-CD45-AF700 (2D1), anti-CD19-FITC (HIB19), anti-CD3-PerCP-Cy5.5 (SK7), anti-CD4-BV510 (RPA-T4; all BioLegend, San Diego, USA) and anti-CD14-APC (M5E2, BD Biosciences, Heidelberg, Germany). Dead cells were excluded by staining with Zombie-NIR (BioLegend, San Diego, USA) (Figure 7). Finally, cells were washed twice with PBS and fixed with PBS+1 % paraformaldehyde overnight. Cells were finally washed twice with PBS+5 % FCS, resuspended in 200 µl PBS+5 % FCS and immediately analyzed using BD LSRFortessa (BD Biosciences, Heidelberg, Germany). Data analysis was performed using BD FACSDiva Version 8.0. Gating strategy is presented in Fig. 7.

### Detection of anti-Dsg3 IgG by ELISA

The presence of IgG auto-antibody against Dsg1 or Dsg3 in the sera of PV patients was evaluated using anti-Dsg1- and anti-Dsg3-ELISA (Euroimmun, Lübeck, Germany) according to the manufacturer's protocol. Anti-human Dsg3 IgG in mouse sera and presence of human anti-Dsg3 IgG1 or IgG4 in sera of PV patients was evaluated using the same ELISA system with modifications from the manufacturer's protocol for the detection of anti-Dsg3 IgG. Serum samples were incubated on Dsg3-coated plates of anti-Dsg3-ELISA (Euroimmun, Lübeck, Germany). Mouse anti-Dsg3 IgG was detected with rabbit anti-mouse-IgG conjugated with HRP (1:2000; Dako, Denmark). Human anti-Dsg3 IgG1 and IgG4 antibodies were detected by mouse anti-human IgG1 or IgG4 detection antibodies (1:8000; BD Biosciences, Germany) prior to incubation with rabbit anti-mouse-IgG conjugated with HRP 388 (1:2000; Dako, Denmark).

### HLA-DRB1*04:02-transgenic mice

HLA-DRA1*01:01-DRB1*04:02/-DQA1*03:01, -DQB1*03:02 (DQ8)-transgenic C57BI6J mice from the inventor's laboratory which expressed the human CD4 co-receptor and were I-Aβ-deficient (I-Aβ-/-), were immunized by i.p. injection with recombinant human Dsg3₍₁₋₅₆₆₎ (SEQ ID NO:9) or respective peptides (SEQ ID NO: 5 to 8) (20- 40 µg) in aluminum hydroxide adjuvant 1% (alum) on days 0 and 14. Final blood samples were taken on day 28 to analyze anti-Dsg3 IgG by ELISA as described above. All animal experiments were reviewed and approved by the local Laboratory Animal Ethics Committees (AZ 397 G50/2020). The experiments were done in compliance with local policies and guidelines on the use of laboratory animals.

### Indirect immunofluorescence

Indirect immunofluorescence with serum samples of immunized mice (diluted at 1:25) was performed on monkey esophagus, according to the manufacturer's instructions (The Binding Site, Birmingham, U.K.), and modified by using a goat anti-mouse IgG AF488-labeled antibody (Invitrogen).

### Keratinocyte dissociation assay

Serum IgG from Dsg3-peptide immunized mouse was initially purified using Protein G Sepharose 4 Fast Flow (Sigma Aldrich, Taufkirchen, Germany). In detail, serum was diluted at 1:1 in 20 mM sodium phosphate buffer (Na₂HPO₄, NaH₂PO₄; pH 7.2) and cleared by centrifugation. Subsequently, the serum was loaded onto the column and rotated over night at 4 °C. Following several washing steps with 20 mM sodium phosphate buffer (pH 7.2), the antibody was eluted into 20 µl 2 M sodium carbonate buffer (Na₂CO₃, NaHCO₃) using 20 mM sodium citrate buffer (Na₃C₆H₅O₇, C₆H₈O₇; pH 2.4). The protein amount was measured by Bradford assay. Depending on the protein amount, fractions were either used directly or concentrated by centrifugation at 5000 rpm for 30 min using an Amicon Ultra-15 centrifugal filter device (Merck, Darmstadt, Germany). To perform the keratinocyte dissociation assay, immortalized human keratinocytes (HaCaT) cells were seeded in a 24 well plate and grown until confluence. Subsequently, cells were treated with either AK23 (75 µg/mL, provided by Dr. Amagai, Keio University Tokyo, Japan and Dr. Eliae Mueller, University Bern, Switzerland), IgG from sera of Dsg3-peptide immunized mice (75 µg/mL) or control-IgG (75 µg/mL) for 24 hours at 37 °C and 8 % CO₂. Cells were washed with HBSS (Hanks balanced salt solution; Gibco, Paisley, Scotland, UK) and incubated with 2.5 U/mL Dispase II (Roche, Basel, Switzerland) solution until the monolayer was completely detached. Subsequently, the cells were incubated with thiazolyl blue tetrazolium bromide (MTT) (5 mg/mL) for 10-15 min at 37°C. For fragmentation, the monolayer was disrupted by mechanical stress in terms of multiple pipetting. To evaluate the number of repetitions necessary for fragmentation, the positive control was pipetted first. The following samples were pipetted with an equal number of repetitions as used for the positive control. The fragments were fixed using 4 % paraformaldehyde and counted afterwards.

### Statistical analysis

Statistical analysis was performed using GraphPad Prism 6.02 (GraphPad Software Inc., La Jolla, USA). Cumulative data are displayed as box plots with median. Normal distribution was tested using the D'Agostino_pearson omnibus normaility test. For group comparisons of non-normally distributed data, the Kruskal-Wallis one way ANOVA-test followed by Dunn's multiple comparisons test was used. Statistical significance for unmatched pairwise comparisons was tested using the Mann-Whitney test. Differences between the groups were considered statistically significant at p values of * < 0.05, ** < 0.01, *** < 0.001.

## Claims

1. An *in vitro* method for diagnosis of pemphigus vulgaris in a patient and/or of determining disease activity in a pemphigus vulgaris patient, the method comprising determination of T cells specific for the extracellular domain of desmoglein 3 (Dsg3-specific T-cells) or for specific epitopes of said extracellular Dsg3 domain in serum of said patient by HLA-class II-dextramer staining.

2. The method of claim 1, wherein the Dsg3-specific T cells are peripheral CD4⁺ T-cells.

3. The method of claim 1 or 2, additionally comprising determination of the cytokine profile of said Dsg3-specific T cells by ELISpot analysis.

4. The method of any of claims 1 to 3, wherein the Dsg3-specific T cells are specific for the Dsg3₍₁₋₅₆₆₎ extracellular domain having the sequence shown in SEQ ID NO:9, wherein one to three amino acids may be deleted, added or substituted by other amino acids.

5. The method of any of claims 1 to 4, wherein the Dsg3-specific T cells are specific for the Dsg3 epitopes Dsg3₍₁₉₀₋₂₀₄₎ having SEQ ID NO:1, Dsg3₍₂₀₆₋₂₂₀₎ having SEQ ID NO:2, Dsg3₍₂₅₁₋₂₆₅₎ having SEQ ID NO: SEQ ID NO:3, Dsg3₍₃₇₅₋₃₉₁₎ having SEQ ID NO:4, Dsg3₍₂₅₄₋₂₆₈₎ having SEQ ID NO:7 and/or Dsg3₍₃₇₈₋₃₉₂₎ having SEQ ID NO: 8, wherein, in said sequences, one to three amino acids may be deleted or added or substituted by other amino acids, respectively.

6. The method of any of claims 1 to 5, wherein the Dsg3-speciifc cells are specific for the Dsg3 epitopes Dsg3₍₂₀₆₋₂₂₀₎ having SEQ ID NO: 2 and Dsg3₍₃₇₈₋₃₉₂₎ having SEQ ID NO:8.

7. The method of any of claims 1 to 6, additionally comprising a step of determining if the patients carry the HLA-class I alleles HLA-DRB1*04:02 and/or HLA-DQB1*05:03.
